# EUROPÄISCHE PATENTSCHRIFT

(11) **EP 3 764 843 B1**
(45) Veröffentlichungstag und Bekanntmachung des Hinweises auf die Patenterteilung: **30.11.2022**
(21) Anmeldenummer: 19709741.3
(22) Anmeldetag: 14.03.2019
(51) Int. Cl.: A47B 81/00, B01L 1/02, C12M 1/00, E05B 65/00, E05C 19/16

(54) **LABORSCHRANKVORRICHTUNG ZUM LAGERN VON LABORPROBEN MIT MAGNETVERSCHLUSS**
LABORATORY CABINET FOR STORING LABORATORY SAMPLES WITH MAGNETIC CLOSURE
DISPOSITIF FORMANT ARMOIRE DE LABORATOIRE DESTINÉ AU STOCKAGE DES ÉCHANTILLONS DE LABORATOIRE À FERMETURE MAGNÉTIQUE

(30) Priorität: 16.03.2018 EP 18162376
(43) Veröffentlichungstag der Anmeldung: 20.01.2021
(73) Patentinhaber: Eppendorf SE, 22339 Hamburg (DE)
(72) Erfinder: FITZER, Jan, 22339 Hamburg (DE); BECHMANN, Gregor, 22339 Hamburg (DE); SCHAFRINSKI, Arne, 22339 Hamburg (DE); MENSCH, Sören, 22339 Hamburg (DE)
(74) Vertreter: Ricker, Mathias
(86) Internationale Anmeldenummer: PCT/EP2019/056466
(87) Internationale Veröffentlichungsnummer: WO 2019/175337

(56) Entgegenhaltungen:
- EP-A1- 0 238 313
- WO-A1-2013/057344
- GB-A- 1 009 996
- GB-A- 2 395 744
- US-A- 2 812 203

## Beschreibung

Die Erfindung betrifft eine Laborschrankvorrichtung zum Lagern von Laborproben mit Magnetverschluss für die Türe. Sie betrifft insbesondere einen Temperierschrank für das Temperieren von Laborproben, insbesondere einen Inkubator für das Wachstum von Zellkulturen. Dokument EP0238313 A1 offenbart eine Laborschrankvorrichtung nach dem Oberbegriff des Anspruchs 1.

Mit solchen Inkubatoren werden in biologischen und medizinischen Laboratorien Zellen in Zellkultur unter kontrollierten Umgebungsbedingungen gehalten, und so das Wachstum lebender Zellen *in vitro* ermöglicht. Dazu werden die Temperatur und die Gaszusammensetzung bzw. die Luftfeuchtigkeit der Atmosphäre im Inneren einer von der Umgebung isolierten Inkubatorkammer durch die apparativen Einrichtungen des Inkubators auf den gewünschten Werten gehalten. Eukaryotische Zellen müssen in CO₂-Inkubatoren kultiviert werden. Die Atmosphäre wird durch Luft mit einem bestimmten CO₂- und O₂-Gehalt und einer bestimmten Luftfeuchtigkeit gebildet, eine geeignete Temperatur ist oftmals 37 °C. Solche Temperierschränke weisen ein die Inkubatorkammer umgebendes Gehäuse auf, beispielsweise ein Außengehäuse, mit einer Gehäuseöffnung, durch die der Benutzer die Proben im Gehäuseinneren, insbesondere in der Inkubatorkammer, lagert und wieder entnimmt. Die Gehäusetüre soll das Gehäuseinnere, in dem die Inkubatorkammer liegt, zuverlässig schließen. Zu diesem Zweck sind im Stand der Technik verschiedene technische Lösungen bekannt. Mechanisch wirkende Verschlüsse weisen beispielsweise einen Riegelverschluss, einen Spann- oder Rastverschluss auf, die zum Öffnen entriegelt werden müssen. Solche mechanischen Lösungen sind aufwändig und erzeugen mechanische Vibrationen am Gehäuse, die sich bis in die Kammer, in der die Proben (eukaryontische Zellen) lagern, fortsetzten. Magnetisch wirkende Verschlüsse, wie sie insbesondere von Kühlschrankverschlüssen bekannt sind, erzeugen beim Zuklappen bzw. beim Öffnen der Türe durch eine hohe magnetische Haftkraft eine mechanische Erschütterung, die sich ebenfalls bis in die Kammer fortsetzen kann. Da Laborproben oftmals empfindlich sind und einen hohen Wert aufweisen, ist es wünschenswert, dass das Öffnen und Schließen der Gehäusetüre eine mechanische Erschütterung oder Vibrationen des Gehäuses vermeidet, da sich diese bis in die Inkubatorkammer mit den sensiblen Proben fortsetzen können.

Der vorliegenden Erfindung liegt deshalb die Aufgabe zugrunde, eine verbesserte Laborschrankvorrichtung bereitzustellen.

Die Erfindung löst diese Aufgabe durch die Laborschrankvorrichtung gemäß Anspruch 1. Bevorzugte Ausgestaltungen sind insbesondere Gegenstände der Unteransprüche.

Die erfindungsgemäß magnetisch wirkende Halteeinrichtung zum Halten der Gehäusetüre in der Verschlussposition bietet den Vorteil, dass aufgrund der berührungsfreien Anordnung des ersten und zweiten Halteelements deren Haftung vermieden wird und deshalb beim Öffnen der Gehäusetüre keine oder nur eine geringe Erschütterung bzw. kein oder nur ein geringer Ruck erzeugt wird. Dadurch wird auf die im Gehäuseinneren, insbesondere in der Inkubatorkammer befindlichen Laborproben keine Erschütterung übertragen, was eine Nutzung der Laborschrankvorrichtung unter störungsfreier Lagerung der Laborproben ermöglicht. Gleichzeitig profitiert die Bedienbarkeit der Laborschrankvorrichtung von den Eigenschaften des magnetischen Feldes. Die Magnetkraft ist etwa dem inversen Abstandsquadrat der miteinander wirkenden magnetischen Partner proportional. In einer bestimmten Relativposition der Gehäusetüre zum Gehäuse bzw. des ersten und zweiten Halteelements wird die Magnetkraft die Gehäusetüre selbsttätig schließen und zuverlässig in die Verschlussposition überführen, ohne dass der Benutzer diese Bewegung sicherstellen muss und ohne dass der Benutzer -oder eine Mechanik- einen etwaigen separaten Verriegelungsvorgang bewirken muss. Da sich die magnetischen Elemente nicht berühren, wird das ruckartige Öffnen von aneinander haftenden Magneten vermieden, das man bei einigen Magnetverschlüssen des Stands der Technik findet. Die Halteeinrichtung ersetzt insbesondere mechanische Verschlusseinrichtungen bzw. Verriegelungen. Durch die berührungslose Wirkung wird ein auf Berührungen beruhender Verschleiß der Halteeinrichtung vermieden. Durch diese Eigenschaften ist die Bedienbarkeit der Gehäusetüre und damit der Laborschrankvorrichtung besonders sicher und komfortabel. Die Halteeinrichtung kann optimal in die Laborschrankvorrichtung integriert werden, da der Platzbedarf der Halteeinrichtung an der Laborschrankvorrichtung relativ gering ist.

Die Laborschrankvorrichtung zum Lagern von Laborproben ist insbesondere ein Temperierschrank zum Temperieren von Laborproben, insbesondere ein CO₂ Inkubator. Solche Geräte werden elektrisch betrieben und weisen einen Spannungsanschluss auf. Die Halteeinrichtung arbeitet vorzugsweise mit einem oder mehreren Permanentmagneten, so dass die Halteeinrichtung unabhängig von der Spannungsversorgung ist. Der Verschluss der Gehäusetüre ist deshalb auch bei einem Ausfall der Spannungsversorgung gewährleistet.

Der Temperierschrank temperiert die Laborproben, das heißt, er hält das Gehäuseinnere und damit die dort lagernden Laborproben im Rahmen von Toleranzen durch eine Temperaturregelung auf einer insbesondere vom Benutzer einstellbaren Solltemperatur. Diese kann über der Raumtemperatur (Umgebungstemperatur) liegen, wie dies bei einem Wärmeschrank oder Inkubator der Fall ist, oder kann unter der Raumtemperatur liegen, wie dies bei einem Kühlschrank oder Gefrierschrank der Fall ist. Bei einer als Klimaschrank ausgebildeten Laborschrankvorrichtung wird vorzugsweise auch ein im Inneren des Gehäuses vorherrschender Klimaparameter im Rahmen von Toleranzen geregelt. Dieser Klimaparameter kann die Luftfeuchtigkeit sein, und/oder eine Gaskonzentration, z.B. eine CO2, O2 und/oder N2-Konzentration. Ein solcher Klimaschrank ist beispielsweise ein Inkubator für aus lebenden Zellkulturen bestehende Laborproben.

Das Gehäuse der Laborschrankvorrichtung ist vorzugsweise ein äußeres Gehäuse, dessen Gehäusewände mit der Umgebung in Kontakt stehen. Das Gehäuse kann aber auch ein innerhalb eines Außengehäuses liegendes inneres Gehäuse sein. Beispielsweise kann ein Inkubator mindestens eine als inneres Gehäuse dienende Kammer aufweisen, die durch mindestens eine Gehäusetüre bzw. Kammertüre verschließbar ist. Die Gehäusetüre kann entsprechend eine äußere Gehäusetüre sein, die in der Verschlussposition an die Umgebung grenzt, oder kann eine innere Gehäusetüre sein, zu der insbesondere eine zusätzliche äußere Gehäusetüre vorgesehen sein kann, wobei nur letztere in der Verschlussposition an die Umgebung grenzt und die innere Gehäusetüre in der Verschlussposition der äußeren Gehäusetüre in einem Hohlraum zwischen innerem Gehäuse, äußerem Gehäuse und äußerer Gehäusetüre liegt.

Die Gehäusetüre weist insbesondere eine Scharniereinrichtung auf, welche die Gehäusetüre schwenkbar mit dem Gehäuse verbindet. Eine solche Schwenktüre wird durch eine Rotation zwischen einer geöffneten Position und der Verschlussposition bewegt. Die Scharniereinrichtung kann insbesondere an der - im bestimmungsgemäßen Gebrauch der Laborschrankvorrichtung - vertikal orientierten Außenkante eines quaderförmigen Gehäuses liegen, welche an die Gehäuseöffnung angrenzt. Die Bodenplatte eines quaderförmigen Gehäuses ist im bestimmungsgemäßen Gebrauch der Laborschrankvorrichtung horizontal angeordnet, die Seitenwände des Gehäuses sind insbesondere vertikal angeordnet, und die Deckplatte des Gehäuses ist insbesondere der Bodenplatte gegenüberliegend horizontal angeordnet. Die Halteeinrichtung ist vorzugsweise an einer Außenkante des Gehäuses angeordnet, an der nicht die Scharniereinrichtung angeordnet ist, vorzugsweise in Verschlussposition gegenüberliegend der Scharniereinrichtung, also insbesondere an der an die Gehäuseöffnung angrenzenden. Eine Halteeinrichtung kann aber auch alternativ oder zusätzlich an einer anderen Außenkante des Gehäuses angeordnet sein, insbesondere einer horizontal verlaufenden Außenkante.

Die Gehäusetüre kann aber auch eine Schiebetüre sein, die durch eine translatorische Bewegung zwischen einer geöffneten Position und der Verschlussposition bewegt wird. Auch eine gemischt schwenkende/translatorische Bewegung der Gehäusetüre ist möglich.

Die Laborschrankvorrichtung kann mehr als eine Halteeinrichtung aufweisen, die jeweils, wie definiert, die in Verschlussposition der Gehäusetüre magnetisch miteinander wechselwirkenden Halteelemente aufweisen. Im Fall von mehreren Halteeinrichtungen sind diese vorzugsweise in Verschlussposition voneinander beabstandet angeordnet. Sie können insbesondere an derselben Außenkante des Gehäuses oder an unterschiedlichen Außenkanten des Gehäuses angeordnet sein. Durch die Bereitstellung mehrerer Halteeinrichtungen kann das gleichmäßige Schließen der Gehäusetüre und die Verteilung der zwischen Türe und Gehäuse wirkenden Verschlusskräfte optimiert werden.

Vorzugsweise sind die mehreren Halteeinrichtungen in identischer Weise aus mindestens einem ersten und einem zweiten Halteelement bestehend ausgeführt. Es kann aber auch vorgesehen sein, dass die mehreren Halteeinrichtungen in unterschiedlicher Weise aus mindestens einem ersten und einem zweiten Halteelement bestehend ausgeführt sind. Dadurch kann die Verschlusscharakteristik der Gehäusetüre, also die mittels der Halteeinrichtungen bewirkte Kraft zwischen Gehäuse und Gehäusetüre, aufgetragen gegen die Auslenkungsstrecke der Gehäusetüre aus der Verschlussposition, in gewünschter Weise beeinflusst werden.

In der Verschlussposition verschließt die Gehäusetüre das Gehäuseinnere vorzugsweise dicht, was insbesondere durch mindestens eine Dichtungseinrichtung der Gehäusetüre bzw. des Rahmens der Gehäuseöffnung gelingt. Durch die Dichtung wird vorzugsweise eine für die hohen Anforderungen des Inkubierens von lebenden Zellkulturen ausreichende Dichtwirkung erzielt. Da bestimmte Gaskonzentrationen und die Temperatur im Inneren des Inkubators geregelt sind, bewirkt eine gute Dichtung einen kosteneffizienten Betrieb. Die Erfindung betrifft aber grundsätzlich auch Laborschrankvorrichtungen mit einem Gehäuse, das das Gehäuseinnere gegenüber der Umgebung nicht vollständig abdichtet.

Die Gruppe von magnetisch wirkenden Halteelementen weist mindestens drei Halteelemente auf entsprechend Anspruch 1 wie nachfolgend noch erläutert wird. Es ist aber ebenso bevorzugt, dass die Gruppe von magnetisch wirkenden Halteelementen mehr als drei Halteelemente beinhaltet. Die magnetische Wirkung der Halteelemente beruht darauf, dass sie Permanentmagneten sind.

Eine Halteeinrichtung kann mehrere Magnetelemente aufweisen, die die gewünschte Magnetkraft erzeugen, insbesondere 4, 5, 6 Magnetelemente oder eine andere Anzahl mit mehr als drei Permanentmagneten.

Durch eine solche Gestaltung kann die Verschlusscharakteristik präzise eingestellt und mit genau definierten Haltekräften wieder gelöst werden.

Ein Halteelement bzw. Magnetelement ist vorzugsweise ein separates Bauteil, das bei Montage der Laborschrankvorrichtung mit dem Gehäuse und/oder der Gehäusetüre befestigt wird. Ein Halteelement bzw. Magnetelement kann aber auch ein integraler Bestandteil des Gehäuses und/oder der Gehäusetüre sein.

Ein Magnetelement ist ein Permanentmagnet oder weist einen solchen auf. Vorzugsweise weist der Magnet ein Abdeckelement auf, um ihn vor mechanischen Schäden oder vor Korrosion zu schützen.

Vorzugsweise ist der Permanentmagnet aus einer Samarium-Cobalt-Legierung hergestellt oder weist diese Materialien auf. Diese Materialien haben sich in feuchten bzw. in mit Chemikalien behafteten Laborumgebungen als besonderes widerstandsfähig gegen Korrosion erwiesen. Es ist aber auch möglich, andere Permanentmagneten zu verwenden, beispielsweise Neodym-Eisenmagnete.

Vorzugsweise weist die Laborschrankvorrichtung mindestens ein elastisches Element auf, das in der Verschlussposition zwischen Gehäuse und Gehäusetüre angeordnet und insbesondere in der Verschlussposition zwischen Gehäuse und Gehäusetüre durch die Haltekraft der Halteeinrichtung komprimiert ist. Das elastische Element dient insbesondere als Anschlag für die Gehäusetüre am Gehäuse, der den Kontakt der Gehäusetüre am Gehäuse mechanisch abfedert und dämpft. Zudem dient das elastische Element als Widerlager für die Haltekraft der Halteeinrichtung, die das elastische Element in der Verschlussposition komprimiert. Die Halteeinrichtung der erfindungsgemäßen Laborschrankvorrichtung bietet bei Verwendung mit einem elastischen Element den Vorteil, dass durch die Reichweite der Magnetkraft Fertigungsschwankungen der Abmessungen oder der Elastizität des elastischen Elements ausgeglichen werden, so dass eine gewisse Unabhängigkeit der Haltekraft und damit der Zuverlässigkeit des Verschlusses von diesen Eigenschaften des elastischen Elements erreicht wird.

Das elastische Element ist insbesondere eine Dichtung, die in der Verschlussposition das Gehäuseinnere hermetisch von der Umgebung abtrennt. Die Dichtung ist insbesondere an einer Außenwand des Gehäuses angeordnet, welche auch die Gehäuseöffnung aufweist. Die Dichtung läuft vorzugsweise durchgehend um die Gehäuseöffnung um. Alternativ oder zusätzlich kann eine solche Dichtung an der Innenseite der Gehäusetüre so angeordnet sein, dass sie die in der Verschlussposition um die Gehäuseöffnung durchgehend umläuft. Die Dichtung weist vorzugsweise Silikon auf bzw. besteht vorzugsweise aus Silikon. Das Silikon kann ein Silikonschaum sein und/oder Hohlräume und/oder Aussparungen aufweisen. Durch solche Poren bzw. Hohlräume wird die gewünschte Elastizität bzw. die thermische Isolierfähigkeit der Dichtung erreicht.

Die Verschlussposition ist insbesondere dadurch gekennzeichnet, dass das Gehäuseinnere durch die Gehäusetüre verschlossen wird, insbesondere dicht verschlossen wird, und insbesondere mittels einer Dichtung für die Zwecke des Inkubierens lebender Zellkulturen verschlossen wird. In der Verschlussposition wird das elastisch verformbare Element bzw. die die elastisch verformbare Dichtung durch die magnetische Haltekraft der Halteeinrichtung elastisch verformt.

Das erste und das zweite Halteelement sind so angeordnet und dazu eingerichtet, die Gehäusetüre in der Verschlussposition durch die Magnetkraft berührungsfrei zu halten. Das erste und das zweite Halteelement berühren sich in der Verschlussposition nicht.

Das erste und zweite Halteelement sind in der Verschlussposition berührungsfrei angeordnet.

Im Falle zweier miteinander wechselwirkender Permanentmagneten kann die Magnetkraft je nach Polung und Ausrichtung der Permanentmagneten zueinander anziehend oder abstoßend sein. Die Magnetkraft zwischen den magnetischen Partnern hängt nicht nur von den jeweiligen Festkörpereigenschaften bzw. magnetischen Feldern ab, sondern auch von der geometrischen Lage der magnetischen Partner zueinander. Die vorliegende Erfindung macht sich dies zunutze, um durch die Positionierung und Ausrichtung der magnetischen Partner zueinander, insbesondere in Verschlussposition, die gewünschte Magnetkraft zu erzielen. Wenn sich die Gehäusetüre in der Verschlussposition befindet, wird die dann wirkende magnetische Kraft als Verschlusskraft bezeichnet. Diese hält die Gehäusetüre in der Verschlussposition in der gewünschten Weise, also mit der gewünschten Kraft, und wirkt so dem Öffnen entgegen.

Das erste Halteelement weist einen ersten Permanentmagneten auf und das zweite Halteelement ein mit diesem ersten Permanentmagneten magnetisch wechselwirkenden zweiten Permanentmagneten auf. Der erste und der zweite Permanentmagnet sind in der Verschlussposition vorzugsweise mit gleichgerichteter Polung angeordnet, so dass zwischen dem ersten und dem zweiten Permanentmagneten eine anziehende Wirkung besteht. Der erste und der zweite Permanentmagnet können in der Verschlussposition aber auch mit entgegen gerichteter Polung angeordnet sein, so dass zwischen dem ersten und dem zweiten Permanentmagneten eine abstoßende Wirkung besteht.

Das erste und zweite Halteelement sind so gegeneinander angeordnet, dass sie beim Schließen der Gehäusetüre die Verschlussposition durch eine Schließbewegung erreichen, durch die das erste und zweite Halteelement parallel zueinander bewegt werden. Durch diese parallele Bewegung wird eine Berührung vermieden. Zudem lässt sich eine solche parallele oder nahezu parallele Bewegung sowohl bei einer rotierenden als auch bei einer verschiebbaren Gehäusetüre erreichen.

Das erste und das zweite Halteelement weisen einen Permanentmagneten auf, der eine sich entlang einer magnetischen Nord-Süd-Polrichtung erstreckende Magnetachse M aufweist, wobei die Schließbewegung beim Erreichen der Verschlussposition vorzugsweise senkrecht zur Magnetachse verläuft oder die Schließbewegung beim Erreichen der Verschlussposition in einem Winkel α zur Magnetachse verläuft, wobei 70° <= α < 90°, vorzugsweise 80° <= α < 90°, 83° <= α <= 87°, vorzugsweise α = 90°.

Insbesondere kann diese Magnetachse senkrecht zu einer Oberfläche, insbesondere zur größten Oberfläche eines Magnetelements stehen. Durch diese Anordnung kann sich eine Magnetkraft über eine längere Bewegungsstrecke aufbauen. Das Maximum der Magnetkraft wird erreicht, wenn sich die magnetischen Partner, insbesondere das erste und zweite Halteelement, direkt gegenüberliegen, d.h., wenn insbesondere ihre zentralen Bauteilachsen bzw. zentralen Magnetachsen M zusammenfallen.

Vorzugsweise weist die Gruppe der Halteelemente mindestens einen quaderförmigen Permanentmagneten auf, dessen Nord-Süd-Polrichtung (Magnetachse) sich senkrecht zur größten Fläche des quaderförmigen Permanentmagneten erstreckt. Die Magnetachse ist dabei senkrecht oder im genannten Winkel α zu der Schließbewegung, mit der die Gehäusetüre ihre Endposition (Verschlussposition) am Gehäuse erreicht. Die größte Fläche definiert dabei den Bereich, über dem sich mit zunehmendem Schließen eine größer werdende Magnetkraft aufbaut.

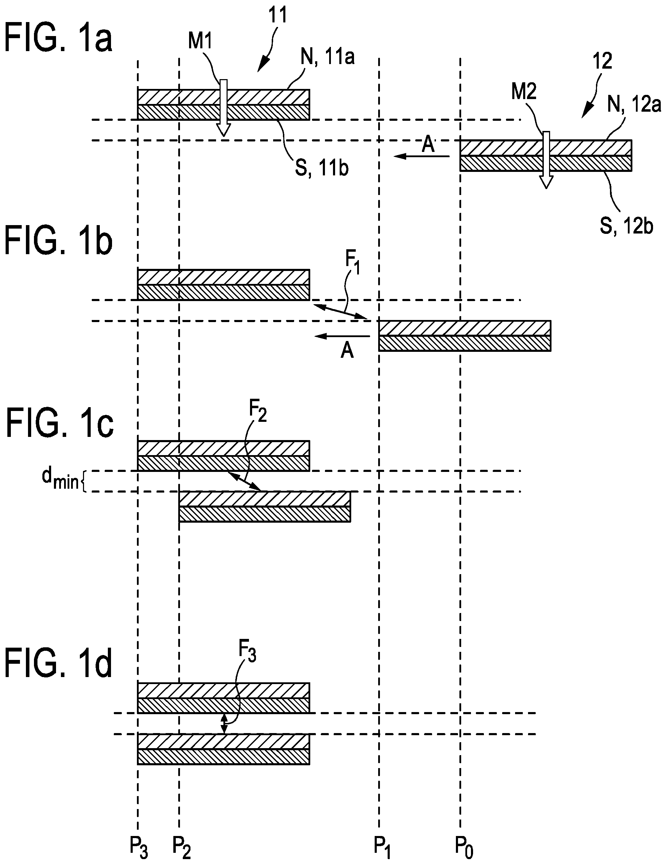

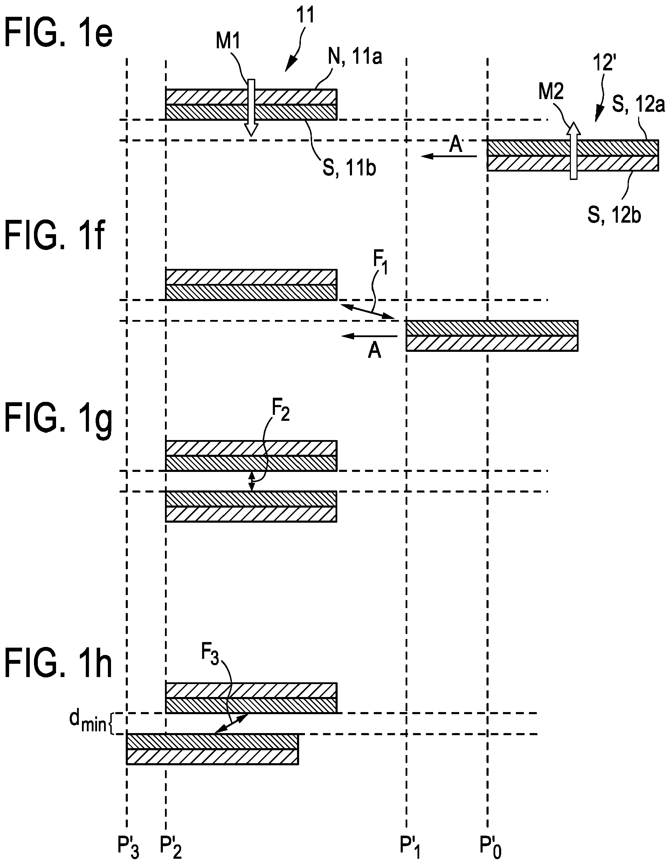

Vorzugsweise ist eine erste Relativposition des ersten und des zweiten Halteelements vorgesehen, in der diese eine erste anziehende Magnetkraft aufeinander ausüben und vorzugsweise ist eine zweite Relativposition des ersten und des zweiten Halteelements vorgesehen, in der diese eine andere, zweite anziehende Magnetkraft aufeinander ausüben, die insbesondere größer ist als die erste Magnetkraft. Das erste und zweite Halteelement sind dann vorzugsweise so gegeneinander angeordnet, dass sie beim Schließen der Gehäusetüre aus der geöffneten Position zunächst die erste Relativposition erreichen, wie dies exemplarisch in Fig. 1a und 1b gezeigt ist, und dann die Verschlussposition erreichen, in der die zweite Relativposition vorliegt, wie dies exemplarisch in Fig. 1c gezeigt ist. Dadurch wird die Gehäusetüre automatisch aus der ersten Relativposition in die zweite Relativposition gezogen.

Vorzugsweise weist die Laborschrankvorrichtung ein elastisches Element auf, das in der Verschlussposition durch die Gehäusetüre mittels dieser Magnetkraft komprimiert wird. Dabei ist vorzugsweise eine dritte Relativposition des ersten und des zweiten Halteelements vorgesehen, in der das elastische Element stärker komprimiert wird als in der zweiten Relativposition, indem das erste und zweite Halteelement in der dritten Relativposition eine dritte anziehende Magnetkraft aufeinander ausüben, die größer ist als die zweite Magnetkraft. Dadurch wird eine Toleranz bei der Dimensionierung der Bauteile der Laborschrankvorrichtung erzielt, da die anziehende Wirkung auch dann gewährleistet ist, wenn das elastische Element bzw. eine Dichtung durch eine Streuung bei der Fertigung oder beim Einbau oder Austausch in die Laborschrankvorrichtung etwas dicker ist als durch die Sollvorgabe definiert. Auch alterungsbedingte Schwankungen des elastischen Elements bzw. einer Dichtung lassen sich so kompensieren.

Vorzugsweise weisen das erste und das zweite Halteelement in der zweiten und dritten Relativposition denselben vorgegebenen Abstand auf. Die in der dritten Relativposition wirkende dritte Magnetkraft ist bei diesem Abstand vorzugsweise die maximal erzielbare Magnetkraft zwischen dem ersten und zweiten Halteelement. Dieses Maximum der Magnetkraft wird insbesondere erreicht, wenn sich die magnetischen Partner, insbesondere das erste und zweite Halteelement, direkt gegenüberliegen, d.h., wenn insbesondere ihre zentralen Bauteilachsen bzw. zentralen Magnetachsen M zusammenfallen. Dies ist anhand der in Fig. 1d exemplarisch gezeigten dritten Relativposition illustriert.

Vorzugsweise ist eine erste Relativposition des ersten und des zweiten Halteelements vorgesehen, in der diese eine erste abstoßende Magnetkraft aufeinander ausüben und vorzugsweise ist eine zweite Relativposition des ersten und des zweiten Halteelements vorgesehen, in der diese eine zweite abstoßende Magnetkraft aufeinander ausüben, die insbesondere größer ist als die erste Magnetkraft, und vorzugsweise ist eine dritte Relativposition des ersten und des zweiten Halteelements vorgesehen, in der diese eine dritte abstoßende Magnetkraft aufeinander ausüben, die insbesondere kleiner ist als die zweite Magnetkraft. Vorzugsweise sind das erste und zweite Halteelement so gegeneinander angeordnet, dass sie beim Schließen der Gehäusetüre zunächst die erste Relativposition erreichen, im weiteren Verlauf der Schließbewegung dann die zweite Relativposition und danach die Verschlussposition erreichen, in der die dritte Relativposition vorliegt. Durch diese Anordnung wird die Gehäusetüre, vorzugsweise in Gegenwart des elastischen Elements zwischen Gehäusetüre und Gehäuse, durch eine abstoßende Magnetkraft in die Verschlussposition gedrängt und auf diese Weise in der Verschlussposition gehalten. Eine Ausführungsform mit einer Haltekraft basierend auf abstoßenden Magnetkräften ist anhand der Figuren 1e bis 1h beispielhaft illustriert.

Vorzugsweise weist die Gruppe der Halteelemente mindestens einen ringförmigen oder hohlzylinderförmigen Permanentmagneten auf, dessen Nord-Süd-Polrichtung (erste Magnetachse) sich senkrecht zur zentralen, durch die Öffnung des ringförmigen oder hohlzylinderförmigen Permanentmagneten verlaufenden geometrischen Achse erstreckt. Die geometrische Achse ist insbesondere die Symmetrieachse des ringförmigen oder hohlzylinderförmigen Permanentmagneten. Der magnetische Partner dieses ringförmigen oder hohlzylinderförmigen Permanentmagneten ist vorzugsweise ein zweiter ringförmiger oder hohlzylinderförmiger Permanentmagnet, der in die Öffnung des ersten ringförmigen oder hohlzylinderförmigen Permanentmagneten einschiebbar ist- Die Nord-Süd-Polrichtung (zweite Magnetachse) des zweiten Permanentmagneten verläuft dabei parallel der ersten Magnetachse. Abhängig davon, ob die Polung des ersten und zweiten Permanentmagneten gleichgerichtet ist oder entgegengerichtet ist, ergibt sich durch die koaxiale und zentrierte Lage des ersten und zweiten Permanentmagneten, in der diese vollständig ineinandergeschoben sind, eine Ruhelage oder ein Umkehrpunkt.

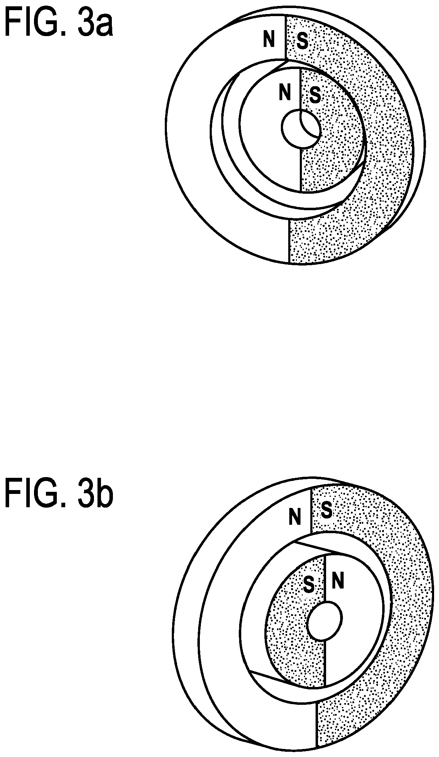

Sind die magnetischen Achsen des ersten und zweiten ringförmigen oder hohlzylinderförmigen Permanentmagneten gleichgerichtet, wie dies exemplarisch in Fig. 3a gezeigt ist, kommt es beim Zusammenschieben des ersten und zweiten ringförmigen oder hohlzylinderförmigen Permanentmagneten entlang der geometrischen Achse zunächst zu einer Abstoßung, dann zu einem Umkehrpunkt und dann zu einer Anziehung, bis die geometrisch zentrierte Lage erreicht ist - dort liegt eine Ruhelage vor. Eine weitere Auslenkung aus dieser Ruhelage in dieselbe Richtung entlang der geometrischen Achse erfolgt dann zunächst gegen die anziehende Kraft und würde dann ab einem weiteren Umkehrpunkt durch Abstoßung beschleunigt. Eine Halteeinrichtung kann so gestaltet sein, dass die Verschlussposition der geometrisch zentrierten Lage entspricht oder einer Lage, in der der zweite Permanentmagnet teilweise mit dem ersten Permanentmagneten in axialer Schieberichtung überlappt und sich die Permanentmagneten anziehen.

Sind die magnetischen Achsen des ersten und zweiten ringförmigen oder hohlzylinderförmigen Permanentmagneten entgegengerichtet, wie dies exemplarisch in Fig. 3b gezeigt ist, kommt es beim Zusammenschieben des ersten und zweiten ringförmigen oder hohlzylinderförmigen Permanentmagneten entlang der geometrischen Achse zunächst zu einer in dieser Richtung zunehmenden Abstoßung, bis die geometrisch zentrierte Lage erreicht ist - dort liegt ein Umkehrpunkt vor. Eine weitere Auslenkung aus dieser Umkehrpunktlage in dieselbe Richtung entlang der geometrischen Achse erfolgt dann zunächst beschleunigt durch die abstoßende, in dieser Richtung abnehmende Kraft. Eine Halteeinrichtung kann so gestaltet sein, dass die Verschlussposition einer Lage entspricht, in der der zweite Permanentmagnet den Umkehrpunkt in Schließrichtung überschritten hat und teilweise mit dem ersten Permanentmagneten in axialer Schieberichtung überlappt und sich die Permanentmagneten abstoßen.

Der magnetische Partner des ringförmigen oder hohlzylinderförmigen Permanentmagneten kann jeweils auch ein ferromagnetisches Bauteilsein, sofern das Abstoßungsprinzip zum Einsatz kommt.

Vorzugsweise ist der mindestens ein Permanentmagnet ein Bestandteil eines Halteelements der Gruppe von Halteelementen und insbesondere Bestandteil eines Magnetelements. Dieses Halteelement weist vorzugsweise ein Abdeckelement oder eine Fassung auf, von der der mindestens ein Permanentmagnet teilweise oder vollständig eingefasst ist, wobei vorzugsweise diese Fassung beim Schließen der Gehäusetüre nicht von einem anderen Halteelement mit Druck beaufschlagt wird. Es ist aber auch möglich, dass das Abdeckelement oder die Fassung als Abstandselement dient, das in der Verschlussposition von einem anderen Halteelement mit Druck beaufschlagt wird.

Die Gruppe der Halteelemente weist ein drittes magnetisch wirkendes Halteelement auf, das am Gehäuse oder an der Gehäusetüre angeordnet ist und das mit dem ersten und zweiten Halteelement die Magnetkraft erzeugt, mit der die Gehäusetüre in der Verschlussposition am Gehäuse gehalten wird. Durch ein drittes Halteelement kann die Verschlusscharakteristik in gewünschter Weise beeinflusst werden.

Das erste und das dritte Halteelement sind so voneinander beabstandet und parallel angeordnet sind, dass zwischen ihnen ein Freiraum gebildet wird, in den das zweite Halteelement in der Verschlussposition eingreift. Es ergibt sich der Vorteil einer geringen mechanischen Belastung der Halteelemente beim Schließen und Öffnen. Oder das zweite und das dritte Halteelement sind so voneinander beabstandet und parallel angeordnet sind, dass zwischen ihnen ein Freiraum gebildet wird, in den das erste Halteelement in der Verschlussposition eingreift. Diese Konfiguration hat den Vorteil, dass das in der Verschlussposition jeweils zwischen zwei anderen Halteelementen angeordnete Halteelement eine verschwindende Nettokraft in Richtung der magnetischen Polung erfährt, da es von beiden Seiten entlang der Richtung der magnetischen Polung durch sich neutralisierende Kräfte gehalten wird.

Die Halteelemente sind in Verschlussposition parallel oder nahezu parallel zueinander angeordnet bzw. weisen parallel zueinander verlaufende Außenflächen auf. Die Magnetelemente dieser Halteelemente sind so angeordnet, dass ihre Magnetachsen M einen Winkel β zueinander bilden, wobei β = 90° - a. Der Winkel α wurde oben erläutert. Durch dieses "Anstellen" eines Magnetelements gegenüber dem anderen Magnetelement kann die beim Schließen/Öffnen nahe der Verschlussposition wirkende Magnetkraft bzw. Verschlusskraft in der gewünschten Weise beeinflusst werden.

Weitere bevorzugte Ausgestaltungen der erfindungsgemäßen Laborschrankvorrichtung lassen sich der Beschreibung der Ausführungsbeispiele gemäß den Figuren entnehmen.

Es zeigen:
Fig. 1a, 1b, 1c und 1d zeigen, in unterschiedlichen Relativpositionen, das erste und zweite Halteelement einer Halteeinrichtung, deren Haltekraft auf anziehenden Magnetkräften beruht und die bei einer erfindungsgemäßen Laborschrankvorrichtung gemäß eines Ausführungsbeispiels einsetzbar ist.
Fig. 1e, 1f, 1g und 1h zeigen, in unterschiedlichen Relativpositionen, das erste und zweite Halteelement einer Halteeinrichtung, deren Haltekraft auf abstoßenden Magnetkräften beruht und die bei einer erfindungsgemäßen Laborschrankvorrichtung gemäß eines weiteren Ausführungsbeispiels einsetzbar ist.
Fig. 2a, 2b, 2c und 2d zeigen jeweils, in einem horizontalen Querschnitt, eine erfindungsgemäße Laborschrankvorrichtung gemäß einem Ausführungsbeispiel, bei dem das erste und zweite Halteelement in den bereits in den Fig. 1a, 1b, 1c und 1d Relativpositionen gezeigt ist.
Fig. 3a und 3b zeigen jeweils Halteeinrichtungen, die nicht unter die beanspruchte Erfindung fallen.
Fig. 4 zeigt, in einem Ausschnitt, eine perspektivische Außenansicht einer Halteeinrichtung an einer erfindungsgemäßen Laborschrankvorrichtung gemäß einem Ausführungsbeispiel, in der Verschlussposition der Gehäusetüre.
Fig. 5 zeigt die Halteeinrichtung der Fig. 4 von einem innerhalb des Gehäuseinneren gelegenen Blickpunkt, bei dem die Gehäusewände ausgeblendet sind.
Fig. 6a zeigt eine perspektivische Außenansicht einer Halteeinrichtung gemäß einem weiteren Ausführungsbeispiel, in der geöffneten Position der Gehäusetüre.
Fig. 6b zeigt die Halteeinrichtung der Fig. 6a im Querschnitt, in der geöffneten Position der Gehäusetüre.
Fig. 6c zeigt die Halteeinrichtung der Fig. 6b im Querschnitt, in der Verschlussposition.
Fig. 6d zeigt die relativen Positionen der im Beispiel der Fig. 6a bis 6c verwendeten Permanentmagneten mit den Magnetachsen M1, M2 und M3 in Bezug auf die Bewegungsrichtung A beim Schließen der Gehäusetüre bzw. des Verschlusses, anhand der Winkel α und β.
Fig. 7a zeigt eine perspektivische Außenansicht einer Halteeinrichtung, die nicht unter die beanspruchte Erfindung fällt, in der geöffneten Position der Gehäusetüre.
Fig. 7b zeigt eine zweite perspektivische Außenansicht der Halteeinrichtung der Fig. 7a, in der geöffneten Position der Gehäusetüre.
Fig. 7c zeigt einen Querschnitt der Halteeinrichtung der Figuren 7a und 7b, in der geöffneten Position der Gehäusetüre.
Fig. 8 zeigt ein Kraft-Weg-Diagramm, in dem die Kraft-Weg-Kurven zu den Ausführungsbeispielen der Fig. 6a bis 6c als "Beispiel 1" gezeigt sind, die Kraft-Weg-Kurven zu den Ausführungsbeispielen der Fig. 7a bis 7c als "Beispiel 2", und ein Vergleichsbeispiel mit einem herkömmlichen Haftmagnetenverschluss.
Fig. 9a zeigt, in Querschnittsansicht und in geschlossener und geöffneter Position, einen herkömmlichen Haftmagnetenverschluss, zur Erläuterung der Kurve "Haftmagnet" in Fig. 8.
Fig. 9b zeigt, in Querschnittsansicht und in geschlossener und geöffneter Position, den Verschluss aus Fig. 6a bis 6c, zur Erläuterung der Kurve "Beispiel 1" in Fig. 8.
Fig. 9c zeigt, in Querschnittsansicht und in geschlossener und geöffneter Position, den Verschluss aus Fig. 7a bis 7c, zur Erläuterung der Kurve "Beispiel 2" in Fig. 8.

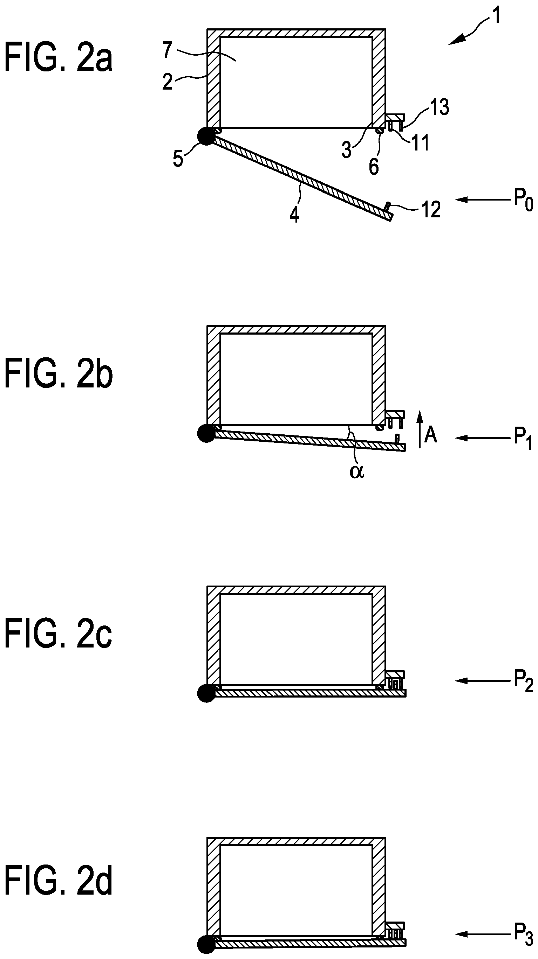

Fig. 1a zeigt das Funktionsprinzip der erfindungsgemäßen Laborschrankvorrichtung für den bevorzugten Fall, dass die in der Verschlussposition wirkende Magnetkraft eine Anziehung des ersten Halteelements 11 und des zweiten Halteelements 12 bewirkt. Dazu weisen der erste und zweite Permanentmagnet dieselbe Polungsrichtung M1=M2 auf, die sich aus derselben relativen Lage von Nordpol N und Südpol S ergibt. Der Nordpol wird durch die Oberseite 12a des plattenförmigen Permanentmagneten 12 gebildet, der Südpol durch dessen Unterseite 12b. Wie anhand der Figuren 2a bis 2d gezeigt ist, ist das erste Halteelement 11 mittels eines Trägerelementes 10 stationär am Gehäuse 2 einer Laborschrankvorrichtung, hier eines Inkubators 1, befestigt, und das zweite Halteelement 12 ist an der Gehäusetüre 4 befestigt, die mittels eines Scharniers 5 an einer benachbart der Gehäuseöffnung 3 gelegenen vertikalen Außenkante des Gehäuses rotierbar gelagert ist. Die Gruppe 11, 12, 13 der Halteelemente weist in Fig. 2a bis 2d ein drittes magnetisch wirkendes Halteelement auf, das in den Figuren 1a bis 1d nicht gezeigt ist. Dieses ist am Gehäuse parallel und in einem Abstand zum ersten Halteelement 11 fest angeordnet, so dass zwischen dem ersten Halteelement 11 und dem dritten Halteelement 13 ein Spalt besteht, in den das zweite Halteelement in der Verschlussposition P2 eingreift. Die drei Halteelemente erzeugen die Magnetkraft, mit der die Gehäusetüre 4 in der Verschlussposition am Gehäuse 2 gehalten wird. Dabei wird die die Gehäusetüre 4 in der Verschlussposition gegen die elastische Dichtung 6 gedrückt. Diese läuft um die Gehäuseöffnung 3 um und wird in der Verschlussposition komprimiert. Das Gehäuseinnere 7 wird dadurch in der Verschlussposition hermetisch abgedichtet. Die Verschlussposition ist die Relativposition P₂.

In der in Fig. 1a gezeigten ersten Relativposition P₀ ist die Gehäusetüre geöffnet. Diese Position wird als Öffnungsposition bezeichnet. Die Halteelemente üben keine wirksame Magnetkraft aufeinander aus.

In der in Fig. 1b gezeigten ersten Relativposition P₁ des ersten Halteelements 11 und des zweiten Halteelements 12 üben diese eine erste anziehende Magnetkraft F₁ aufeinander aus, welche die Gehäusetüre selbstständig, d.d. ohne weitere Benutzereinwirkung in die Verschlussposition bewegt. Die Schließbewegung A, mit der das erste und zweite Halteelement in die Verschlussposition P₂ bewegt werden, ist aufgrund des kleinen Verschlusswinkels a (siehe Fig. 2b) der Gehäusetüre gegenüber der Gehäusefront näherungsweise eine lineare Bewegung A.

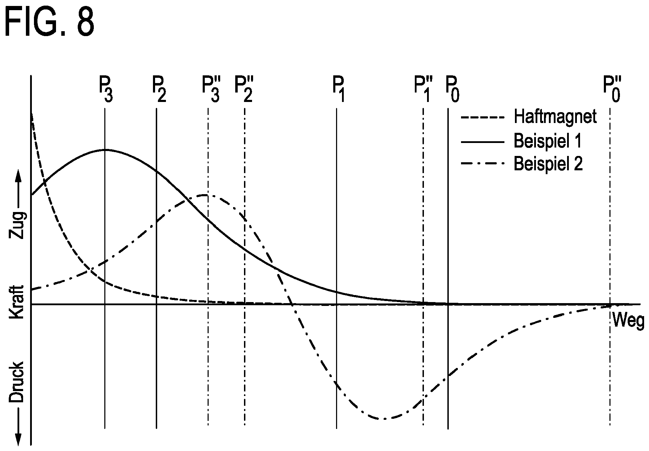

In der in Fig. 1c gezeigten zweiten Relativposition P₂ des ersten Halteelements 11 und des zweiten Halteelements 12 üben diese eine zweite anziehende Magnetkraft F₂ aufeinander aus, welche größer ist als die erste Magnetkraft. In dieser Position ist die Gehäusetüre verschlossen (Verschlussposition) und die Dichtung 6 wird komprimiert, so dass das Gehäuseinnere 7 hermetisch gegen die Umgebung abgedichtet ist. In dieser Relativposition P₂ ist die zentrierte Lage der ersten und zweiten Halteelemente noch nicht erreicht, in der diese zentriert einander gegenüberliegen und die maximale Magnetkraft F₃ wirkt. Diese zentrierte Position P₃ würde erst nach der Relativposition P₂ erreicht, wenn die Schließbewegung A fortgesetzt würde. Da aber in der Relativposition P₂ die einander entgegenwirkende Magnetkraft und die Rückstellkraft des elastisch verformbaren Dichtungselements 6 den gleichen Betrag aufweisen, wird die Position P₃ in der Regel nicht und allenfalls dann erreicht, wenn die Türe manuell oder durch ein Vakuum im Gehäuseinneren stark gegen die Dichtung gepresst wird. Die Verschlussposition kann sich im Laufe der Lebensdauer der Dichtung 6 verlagern, indem die elastische Verformbarkeit der Dichtung alterungsbedingt abnimmt und die Magnetkraft zu einer stärkeren Kompression der Dichtung führt. Das zeigt einen Vorteil der Erfindung, bei der die berührungsfreie bzw. parallele Beweglichkeit des ersten und zweiten Halteelements eine Variabilität der Verschlussposition ermöglicht und somit eine verbesserte Lagetoleranz bewirkt. Die an den Positionen P₀, P₁, P₂ und P₃ wirkenden Magnetkräfte können anhand der Kraft-Weg-Kurve "Beispiel 1" in Fig. 8 eingeschätzt werden.

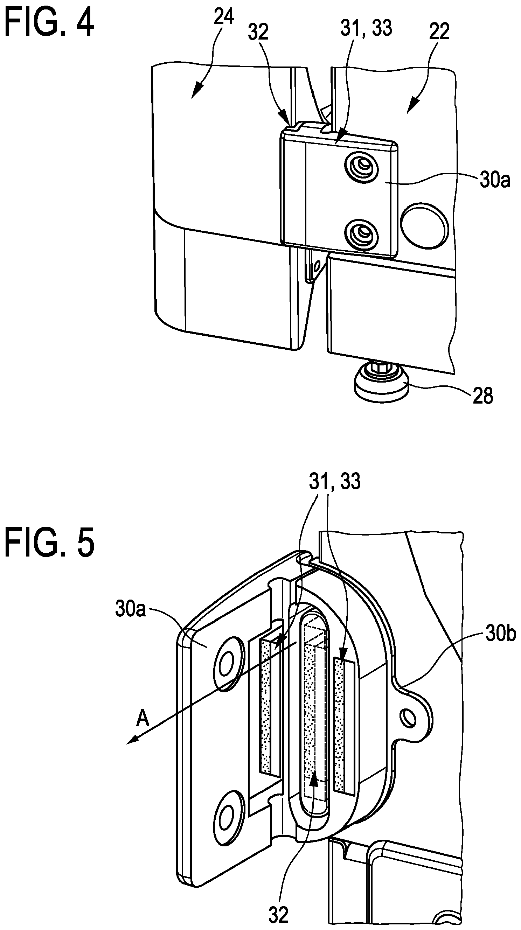

Die Anordnung einer Halteeinrichtung mit erstem, zweitem und dritten Halteelement, die in Fig. 2a gezeigt ist, wird auch im Ausführungsbeispiel der Fig. 4 und 5 umgesetzt. Das erste Halteelement 31 und das dritte Halteelement 33 sind Magnetelemente, hier jeweils bestehend aus einem Permanentmagneten mit gleicher Polungsrichtung, die voneinander beabstandet am Trägerelement 30 befestigt sind. Das Trägerelement 30a ist an der Außenseite des Gehäuses 22 eines Inkubators 21 befestigt. In den zwischen ersten Halteelement 31 und dem dritten Halteelement 33 gebildeten Spalt greift das zweite Halteelement 32 ein, ebenfalls ein Permanentmagnet mit derselben Polung wie der erste und dritte Permanentmagnet, in Verschlussposition betrachtet. Das zweite Magnetelement 32 ist an einem Trägerelement 30b befestigt, das mit der Gehäusetüre 24 verbunden ist.

Eine Ausführungsform der Halteeinrichtung mit einer Haltekraft basierend auf abstoßenden Magnetkräften ist anhand der Figuren 1e bis 1h beispielhaft illustriert. Die Figuren 1e bis 1h zeigen das Funktionsprinzip der erfindungsgemäßen Laborschrankvorrichtung für den bevorzugten Fall, dass die in der Verschlussposition wirkende Magnetkraft eine Abstoßung des ersten Halteelements 11 und des zweiten Halteelements 12' bewirkt. Dazu weisen der erste Permanentmagnet 11 und der zweite Permanentmagnet 12' entgegen gerichtete Polungsrichtungen M1<>M2 auf, die sich aus der Umkehrung der relativen Lage von Nordpol N und Südpol S ergibt, im Vergleich zum Permanentmagneten 12 aus Fig. 1a. Der Südpol wird hier durch die Oberseite 12a des plattenförmigen Permanentmagneten 12' gebildet, der Nordpol durch dessen Unterseite 12b. Hier ist eine erste Relativposition P1' des ersten Halteelements 11 und des zweiten Halteelements 12' vorgesehen, in der diese eine erste abstoßende Magnetkraft F1 aufeinander ausüben. In einer zweiten Relativposition P2' des ersten und des zweiten Halteelements üben diese eine zweite abstoßende Magnetkraft F2 aufeinander aus, die insbesondere größer ist als die erste Magnetkraft F1 und an dem hier gezeigten Umkehrpunkt mit direkt gegenüberliegenden Halteelementen 11, 12' maximal ist (Umkehrpunkt). In der dritten Relativposition P3' des ersten und des zweiten Halteelements üben diese eine dritte abstoßende Magnetkraft F3 aufeinander aus, die kleiner ist als die zweite Magnetkraft F2. Das erste und zweite Halteelement sind so parallel und berührungsfrei gegeneinander angeordnet, dass sie beim Schließen der Gehäusetüre zunächst die erste Relativposition P1' erreichen, im weiteren Verlauf der Schließbewegung dann die zweite Relativposition P2' und danach die Verschlussposition erreichen, in der die dritte Relativposition P3' vorliegt. Durch diese Anordnung wird die Gehäusetüre, vorzugsweise in Gegenwart des elastischen Elements zwischen Gehäusetüre und Gehäuse, durch eine abstoßende Magnetkraft in die Verschlussposition P3' gedrängt und auf diese Weise in der Verschlussposition gehalten.

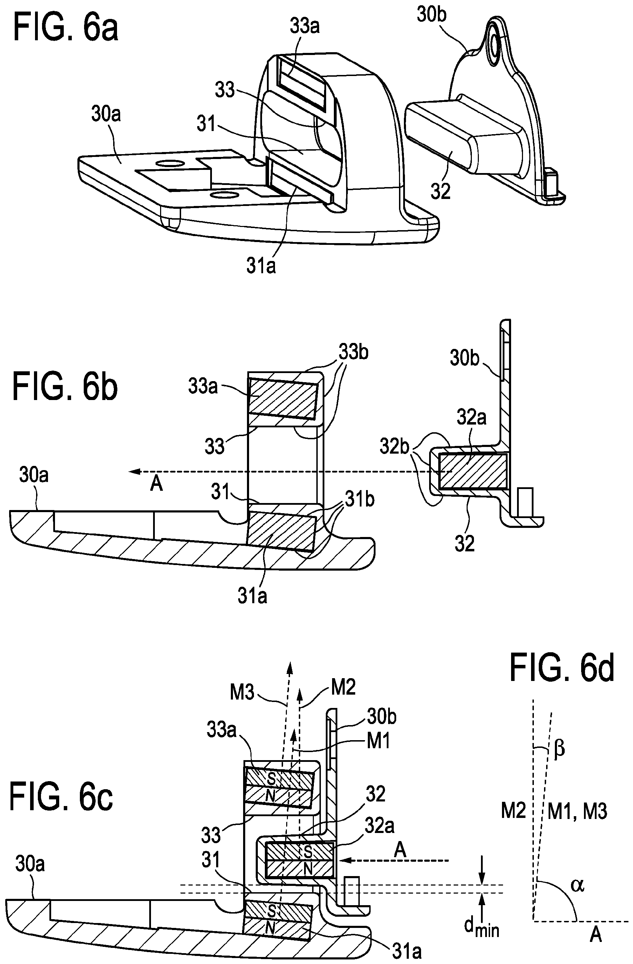

Fig. 6a zeigt eine perspektivische Außenansicht einer Halteeinrichtung gemäß einem weiteren Ausführungsbeispiel, in der geöffneten Position der Gehäusetüre. Der Aufbau dieser Halteeinrichtung und deren Montage an Gehäusetüre und Gehäuse entsprechen den in Fig. 4 und Fig. 5 gezeigten Anordnungen. Die Besonderheit des Beispiels der Fig. 6a bis 6c liegt darin, dass die Permanentmagneten 31a und 33a des ersten und des dritten Halteelements 31, 33 (gehäuseseitig angebracht) gegenüber dem Permanentmagneten 32a des zweiten Halteelements 32 (an der Gehäusetüre angebracht) innerhalb des jeweiligen Halteelements 31, 33 verkippt angeordnet sind. Die Permanentmagneten 31a und 33a sind gegenüber dem Permanentmagneten 32a so verkippt, dass die Magnetachse M1 des ersten Permanentmagneten 31a und die Magnetachse M3 des dritten Permanentmagneten 33a gegenüber der Magnetachse M2 des zweiten Permanentmagneten 32a um den Winkel β = 90° - α, wobei a der Winkel der Magnetachsen M1 und M3 gegenüber der Bewegungsrichtung A ist. Eine Magnetachse verläuft jeweils entlang der Polungsrichtung des Permanentmagneten, die sich vom Nordpol zum Südpol des Permanentmagneten erstreckt.

In Fig. 6c ist der Verschluss so im Querschnitt gezeigt, wie er in einer Aufsicht, oder, ebenso möglich, in Untersicht, am Laborschrank 100 montiert ist. Sämtliche Halteelemente sind hier insbesondere so montiert, dass ihre Magnetachsen M1, M2 du M3 im bestimmungsgemäßen Gebrauch des Laborschranks jeweils senkrecht zur Vertikalen, also zur Gravitationsrichtung, stehen. Die Magnetachsen M1, M2 du M3 könnten aber, insbesondere bei der hier gezeigten verkippten Anordnung der Permanentmagneten, auch senkrecht zu einer anderen Achsrichtung stehen, oder könnten jeweils anders ausgerichtet sein. Durch die Verkippung wird die Kraft-Weg-Kurve in der gewünschten Weise gestaltet, wie dies bei der Kurve zu "Beispiel 1" in Fig. 8 gezeigt ist. Der erste, zweite und dritte Permanentmagnet 31a, 32a, 33a ist hier jeweils als quaderförmiges, plattenartiges Bauteil ausgebildet, bei dem sich die Polungsrichtung senkrecht zur Hauptebene des Bauteils erstreckt, die parallel zu den beiden größten Außenflächen verläuft. Die Magnetachse M1, M2, M3 ist jeweils die Normale zur Hauptebene des jeweiligen quaderförmigen Bauteils.

In Fig. 6b ist gezeigt: das erste Halteelement 31 ist ein im Wesentlichen quaderförmiges Bauteil, das aus einer Fassung 31b besteht, die einen quaderförmigen Hohlraum einfasst, in dem der quaderförmige Permanentmagnet 31a eingefasst und befestigt ist, zum Beispiel durch Verkleben. Das zweite Halteelement 32 ist analog ein im Wesentlichen quaderförmiges Bauteil, das aus einer Fassung 32b besteht, die einen quaderförmigen Hohlraum einfasst, in dem der quaderförmige Permanentmagnet 32a eingefasst und befestigt ist, zum Beispiel durch Verkleben. Das dritte Halteelement 33 ist analog ein im Wesentlichen quaderförmiges Bauteil, das aus einer Fassung 33b besteht, die einen quaderförmigen Hohlraum einfasst, in dem der quaderförmige Permanentmagnet 33a eingefasst und befestigt ist, zum Beispiel durch Verkleben. In Fig 6c ist am besten zu sehen, dass die Außenflächen des ersten, zweiten und dritten Halteelements 31, 32, 33 in der gezeigten Verschlussposition parallel zueinander stehen - vereinfacht ausgedrückt stehen die quaderförmigen bzw- plattenförmigen Halteelemente parallel zueinander, während die Permanentmagneten M1 und M3 gegenüber M2 um denselben Winkel β verkippt sind. Durch die parallelen Außenflächen der Halteelemente wird das Merkmal besonders einfach umsetzbar, dass das erste und zweite -bzw. auch das zweite und dritte- Halteelement so gegeneinander angeordnet sind, dass sie beim Schließen der Gehäusetüre die Verschlussposition durch eine Schließbewegung erreichen, durch die das erste und zweite Halteelement parallel zueinander bewegt werden. Dieses Merkmal lässt sich aber auch erreichen, wenn die Außenflächen der Halteelemente nicht parallel zueinander verlaufen.

Fig. 3a und 3b zeigen Halteeinrichtungen, die nicht unter die beanspruchte Erfindung fallen.

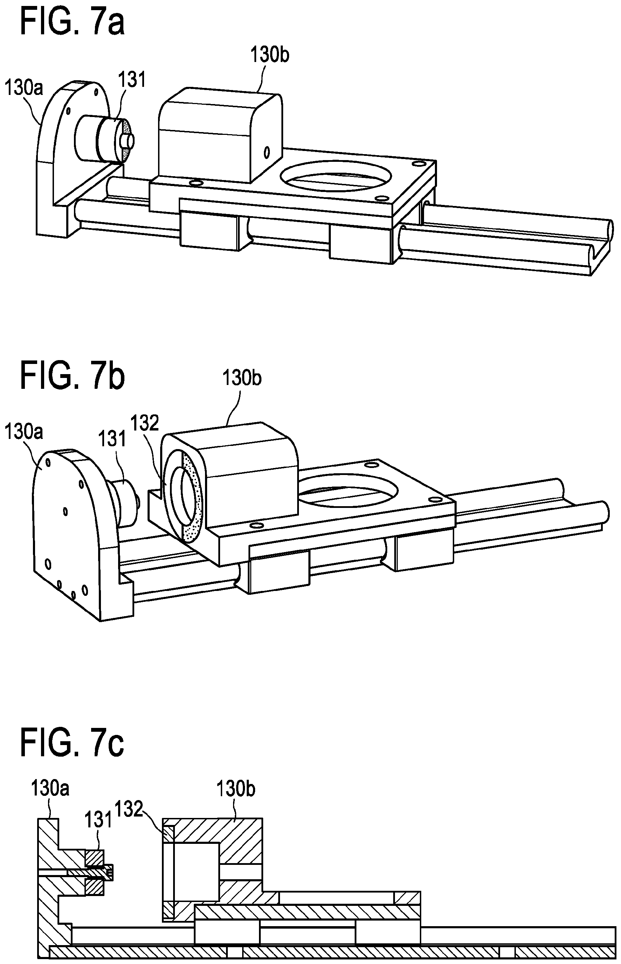

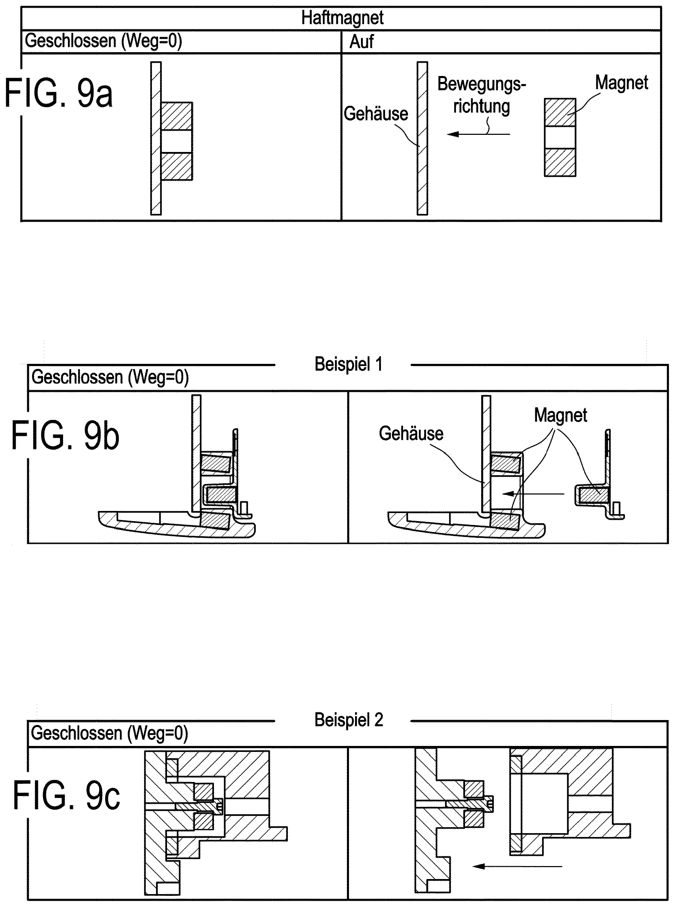

Fig. 7a zeigt eine perspektivische Außenansicht einer Halteeinrichtung die nicht unter die beanspruchte Erfindung fällt, in der geöffneten Position der Gehäusetüre. Das hier als "Beispiel 2" (siehe Fig. 8, 9c) bezeichnete Beispiel folgt dem in Fig. 3a gezeigten Prinzip mit in Verschlussposition konzentrisch ineinandergreifenden, kreisringförmigen Permanentmagneten 131, 132. Gezeigt ist ein Beispiel für eine Schiebetüre, bei der das erste Halteelement 130a am Gehäuse eines Laborschranks (hier nicht gezeigt) angebracht wird und das zweite Halteelement 130b an der Gehäuseschiebetüre (hier nicht gezeigt). Der Schiebemechanismus der Schiebetüre wird hier exemplarisch durch die gezeigte lineare Schienenführung repräsentiert, die im Versuch verwendet wurde, um die Kraft-Weg-Kurve "Beispiel 2" in Fig. 8 zu bestimmen. Beim Schließen dieses Verschlusses aus der geöffneten Position, wie in Fig. 7a bis 7c gezeigt, entsprechend P"₀ in Fig. 8, misst man zunächst eine abstoßende Magnetkraft (P"₁ in Fig. 8). Diese abstoßende Magnetkraft läuft bis zum Umkehrpunkt eines Maximums. Bei weiterer Annäherung der Magnetringe 131, 132 wird ein Punkt passiert, bei dem keine magnetische Wechselwirkung herrscht (Kraft=0), nach dem sofort eine anziehende Wechselwirkung einsetzt, die bis auf ein Maximum (P"₃) läuft. Eine geeignete Verschlussposition ist, analog des Beispiels 1, die Position P"₂, in der die anziehende Kraft noch nicht maximal ist. Dies gibt Reserven für eine Toleranz gegenüber unterschiedlich dicken Türdichtungen, wie bereits erläutert wurde. Die Position P"₃ wird also normalerweise nicht oder allenfalls bei grenzwertig dünnen Dichtungen erreicht. Wie man sieht, wirkt die abstoßende Kraft zwischen P"₀ und P"₂ wie ein Riegel, so dass man das Beispiel 2 auch als magnetischen Riegel bezeichnen kann.

In Fig. 8 ist ebenfalls die Kraft-Weg-Kurve eines herkömmlichen Haftverschlusses gezeigt, bei dem die anziehende Kraft bei Annäherung im Wesentlichen proportional zum inversen Abstandsquadrat verläuft. Auf den letzten Millimetern der Annäherung bis zum Anhaften der Magnete (Fig. 9a) nimmt die Magnetkraft beinahe schlagartig zu, bis auf ein hohes Maximum. Eine solche Anordnung verursacht mechanische Stöße und Vibrationen, die bei den wesentlich sanfter bedienbaren Halteeinrichtungen gemäß der Erfindung vermieden werden können.

## Patentansprüche

1. Laborschrankvorrichtung (1; 21) zum Lagern von Laborproben, aufweisend
ein Gehäuse (2; 22) mit einer Gehäuseöffnung (3), durch die ein Gehäuseinneres (7) des Gehäuses für den Benutzer zugänglich ist,
eine Gehäusetüre (4; 24) zum Schließen der Gehäuseöffnung in einer Verschlussposition (P2; P2'),
eine Halteeinrichtung zum Halten der Gehäusetüre in der Verschlussposition,
wobei die Halteeinrichtung eine Gruppe von magnetisch wirkenden Halteelementen (11, 12,13; 31, 32, 33) aufweist, die ein erstes Halteelement (11; 31) beinhaltet, das am Gehäuse angeordnet ist und ein zweites Halteelement (12; 32) beinhaltet, das an der Gehäusetüre (4) angeordnet ist,
**dadurch gekennzeichnet, dass**
das erste (11; 31) und das zweite (12; 32) Halteelement berührungsfrei angeordnet sind und dazu eingerichtet sind, die Schranktüre in der Verschlussposition durch Magnetkraft zu halten,
das erste (11; 31) und zweite (12; 32) Halteelement so gegeneinander angeordnet sind, dass sie beim Schließen der Gehäusetüre (4; 24) die Verschlussposition durch eine Schließbewegung erreichen, durch die das erste und zweite Halteelement parallel oder nahezu parallel zueinander bewegt werden,
das erste (11; 31) und das zweite (12; 32) Halteelement jeweils einen Permanentmagneten (31a; 32a) aufweisen, der eine sich entlang einer magnetischen Nord-Süd-Polrichtung erstreckende Magnetachse (M1; M2) aufweist, und die Schließbewegung (A) beim Erreichen der Verschlussposition (P2) senkrecht zur Magnetachse verläuft oder die Schließbewegung (A) beim Erreichen der Verschlussposition in einem Winkel α zur Magnetachse verläuft, wobei 70° <= α < 90°, und
die Gruppe der Halteelemente ein drittes (13; 33) magnetisch wirkendes Halteelement aufweist, das einen Permanentmagneten (33a) aufweist und am Gehäuse (4; 24) oder an der Gehäusetüre angeordnet ist und das mit dem ersten (11; 31) und zweiten (12; 32) Halteelement die Magnetkraft erzeugt, mit der die Gehäusetüre in der Verschlussposition am Gehäuse (4; 24) gehalten wird, wobei das erste (11; 31) und das dritte (13; 33) Halteelement so voneinander beabstandet und mit parallel zueinander verlaufenden und in der Verschlussposition (P2; P2') gegenüberliegenden Außenflächen angeordnet sind, dass zwischen ihnen ein Freiraum gebildet wird, in den das zweite (12; 32) Halteelement in der Verschlussposition eingreift, oder wobei das zweite (12; 32) und das dritte (13; 33) Halteelement so voneinander beabstandet und mit parallel zueinander verlaufenden und in Verschlussposition (P2; P2') gegenüberliegenden Außenflächen angeordnet sind, dass zwischen ihnen ein Freiraum gebildet wird, in den das erste (11; 31) Halteelement in der Verschlussposition (P2; P2') eingreift.

2. Laborschrankvorrichtung gemäß Anspruch 1, wobei das erste (11; 31) und zweite (12; 32) Halteelement so gegeneinander angeordnet sind, dass sie beim Schließen der Gehäusetüre zunächst eine erste Relativposition (P1) erreichen und dann die Verschlussposition erreichen, in der eine zweite Relativposition (P2) vorliegt, und
das erste und zweite Halteelement in der ersten Relativposition (P1) eine erste anziehende Magnetkraft (F1) und in der zweiten Relativposition (P2) eine zweite anziehende Magnetkraft (F2) aufeinander ausüben, die größer ist als die erste Magnetkraft.

3. Laborschrankvorrichtung gemäß Anspruch 2, wobei das erste (11; 31) und das (12; 32) zweite Halteelement in einer dritten Relativposition (P3) eine dritte anziehende Magnetkraft (F3) aufeinander ausüben, die größer ist als die zweite Magnetkraft, und die Laborschrankvorrichtung ein elastisches Element (6) aufweist, das in der Verschlussposition durch die Gehäusetüre mittels Magnetkraft komprimiert wird, wobei das elastische Element (6) in der dritten Relativposition (P3) stärker komprimiert wird als in der zweiten Relativposition.

4. Laborschrankvorrichtung gemäß Anspruch 3, wobei das erste (11; 31) und das (12; 32) zweite Halteelement in der zweiten (P2) und dritten (P3) Relativposition denselben vorgegebenen Abstand aufweisen, und die dritte Magnetkraft die bei diesem Abstand maximal erzielbare Magnetkraft zwischen dem ersten (11; 31) und zweiten (12; 32) Halteelement ist.

5. Laborschrankvorrichtung gemäß einem der vorangehenden Ansprüche, wobei die Gruppe der Halteelemente mindestens einen quaderförmigen Permanentmagneten aufweist, dessen Nord-Süd-Polrichtung sich senkrecht zur größten Fläche des quaderförmigen Permanentmagneten erstreckt.

6. Laborschrankvorrichtung gemäß einem der vorangehenden Ansprüche, wobei die Gruppe der Halteelemente mindestens einen ringförmigen oder hohlzylinderförmigen Permanentmagneten aufweist, dessen Nord-Süd-Polrichtung sich senkrecht zur zentralen, durch die Öffnung des ringförmigen oder hohlzylinderförmigen Permanentmagneten verlaufenden Achse erstreckt.

7. Laborschrankvorrichtung gemäß Anspruch 5 oder 6, wobei der mindestens eine Permanentmagnet Bestandteil eines Halteelements der Gruppe von Halteelementen ist, das eine Fassung aufweist, von der der mindestens eine Permanentmagnet eingefasst ist, wobei diese Fassung beim Schließen der Gehäusetüre nicht von einem anderen Halteelement mit Druck beaufschlagt wird.

8. Laborschrankvorrichtung gemäß einem der vorangehenden Ansprüche, der ein Temperierschrank zum Temperieren von Laborproben, insbesondere ein Inkubator für Zellkulturen ist.

9. Laborschrankvorrichtung gemäß einem der vorangehenden Ansprüche, wobei das Gehäuse (4; 24) der Laborschrankvorrichtung ein äußeres Gehäuse ist, dessen Gehäusewände mit der Umgebung der Laborschrankvorrichtung in Kontakt stehen.

10. Laborschrankvorrichtung gemäß einem der vorangehenden Ansprüche 1 bis 5, wobei das Gehäuse (4; 24) der Laborschrankvorrichtung ein innerhalb eines Außengehäuses liegendes inneres Gehäuse ist, wobei die Gehäusewände des Außengehäuses mit der Umgebung der Laborschrankvorrichtung in Kontakt stehen.

11. Laborschrankvorrichtung gemäß einem der vorangehenden Ansprüche, wobei ein Permanentmagnet der Halteeinrichtung eine Samarium-Cobalt-Legierung aufweist oder ein Neodym-Eisenmagnet ist.

12. Laborschrankvorrichtung gemäß einem der vorangehenden Ansprüche 1 bis 6, wobei ein Halteelement (11, 12,13; 31, 32, 33) der Halteeinrichtung eine Fassung (31b; 32b; 33b) aufweist, von der ein Permanentmagnet des Halteelements teilweise oder vollständig eingefasst ist.

## Claims

1. Laboratory cabinet device (1; 21) for storing laboratory samples, comprising
a housing (2; 22) having a housing opening (3) through which a housing interior (7) of the housing is accessible to the user;
a housing door (4; 24) for closing the housing opening in a closing position (P2; P2');
a holding means for holding the housing door in the closing position,
wherein the holding means comprises a group of magnetically acting holding elements (11, 12, 13; 31, 32, 33) including a first holding element (11; 31) arranged on the housing and including a second holding element (12; 32) arranged on the housing door (4),
**characterized in that**
the first (11; 31) and the second (12; 32) holding element are arranged in a contact-free manner and are adapted to retain the housing door in the closing position by magnetic force,
the first (11; 31) and second (12; 32) holding elements are arranged against each other in such a way that, when the housing door (4; 24) is closed, they reach the closing position by a closing movement by which the first and second holding elements are moved parallel or almost parallel to each other
the first (11; 31) and the second (12; 32) holding elements each comprise a permanent magnet (31a; 32a) comprising a magnetic axis (M1; M2) extending along a magnetic north-south pole direction, and the closing movement (A) upon reaching the closing position (P2) is perpendicular to the magnetic axis, or the closing movement (A) upon reaching the closing position is at an angle to the magnetic axis, wherein 70° <= < 90°, and
the group of holding elements comprises a third (13; 33) magnetically acting holding element which comprises a permanent magnet (33a) and is arranged on the housing (4; 24) or on the housing door and which, together with the first (11; 31) and second (12; 32) generating the magnetic force with which the housing door is held in the closing position on the housing (4; 24), wherein the first (11; 31) and third (13; 33) holding elements being spaced apart from one another and having outer surfaces which run parallel to one another and are opposite one another in the closing position (P2; P2') in such a way that a free space is formed between them, in which the second (12; 32) holding element engages in the closing position, or wherein the second (12; 32) and the third (13; 33) holding element are arranged at a distance from one another and with outer surfaces running parallel to one another and opposite one another in the closing position (P2; P2') that a free space is formed between them, in which the first (11; 31) holding element engages in the closing position (P2; P2').

2. Laboratory cabinet device according to claim 1, wherein the first (11; 31) and second (12; 32) holding elements are arranged against each other in such a way that, when the housing door is closed, they first reach a first relative position (P1) and then reach the closing position in which there is a second relative position (P2), and
the first and second holding elements exert a first attractive magnetic force (F1) on each other in the first relative position (P1) and a second attractive magnetic force (F2) on each other in the second relative position (P2), which is greater than the first magnetic force.

3. Laboratory cabinet device according to claim 2, wherein the first (11; 31) and the (12; 32) second holding element exert on each other in a third relative position (P3) a third attracting magnetic force (F3) which is greater than the second magnetic force, and
the laboratory cabinet device comprises an elastic element (6) which is compressed in the closing position by the housing door by means of magnetic force, the elastic element (6) being compressed more strongly in the third relative position (P3) than in the second relative position.

4. Laboratory cabinet device according to claim 3, wherein the first (11; 31) and the (12; 32) second holding elements have the same predetermined distance in the second (P2) and third (P3) relative positions, and the third magnetic force is the maximum achievable magnetic force between the first (11; 31) and second (12; 32) holding elements at this distance.

5. Laboratory cabinet device according to one of the preceding claims, wherein the group of holding elements comprises at least one cuboidal permanent magnet, the north-south pole direction of which extends perpendicularly to the largest surface of the cuboidal permanent magnet.

6. Laboratory cabinet device according to any one of the preceding claims, wherein the group of holding elements comprises at least one annular or hollow cylindrical permanent magnet, the north-south pole direction of which extends perpendicularly to the central axis extending through the opening of the annular or hollow cylindrical permanent magnet.

7. Laboratory cabinet device according to claim 5 or 6, wherein the at least one permanent magnet is a component of a holding element of the group of holding elements, which has a socket by which the at least one permanent magnet is enclosed, wherein this socket is not subjected to pressure by another holding element when the housing door is closed.

8. Laboratory cabinet device according to one of the preceding claims, which is a temperature control cabinet for temperature control of laboratory samples, in particular an incubator for cell cultures.

9. laboratory cabinet device according to one of the preceding claims, wherein the housing (4; 24) of the laboratory cabinet device is an outer housing, the housing walls of which are in contact with the environment of the laboratory cabinet device.

10. Laboratory cabinet device according to any one of the preceding claims 1 to 5, wherein the housing (4; 24) of the laboratory cabinet device is an inner housing lying within an outer housing, the housing walls of the outer housing being in contact with the environment of the laboratory cabinet device.

11. Laboratory cabinet device according to any one of the preceding claims, wherein a permanent magnet of the holding device comprises a samarium-cobalt alloy or is a neodymium-iron magnet.

12. Laboratory cabinet device according to any one of the preceding claims 1 to 6, wherein a holding element (11, 12, 13; 31, 32, 33) of the holding device has a socket (31b; 32b; 33b), by which a permanent magnet of the holding element is partially or completely enclosed.

## Revendications

1. Dispositif d'armoire de laboratoire (1 ; 21) pour conserver des échantillons de laboratoire, présentant
un coffret (2 ; 22) avec une ouverture de coffret (3) à travers laquelle un intérieur de coffret (7) du coffret est accessible à l'utilisateur,
une porte de coffret (4 ; 24) pour fermer une ouverture de coffret dans une position de fermeture (P2 ; P2'),
un équipement de maintien pour maintenir la porte de coffret dans la position de fermeture,
dans lequel l'équipement de maintien présente un groupe d'éléments de maintien (11, 12, 13 ; 31, 32, 33) à action magnétique qui contient un premier élément de maintien (11 ; 31) qui est agencé sur le coffret et qui contient un deuxième élément de maintien (12 ; 32) qui est agencé sur la porte de coffret (4),
**caractérisé en ce que**
le premier (11 ; 31) et le deuxième (12 ; 32) élément de maintien sont agencés sans contact et conçus pour maintenir la porte d'armoire dans la position de fermeture grâce à une force magnétique,
le premier (11 ; 31) et le deuxième (12 ; 32) élément de maintien sont agencés l'un par rapport à l'autre de sorte que, lors de la fermeture de la porte de coffret (4 ; 24), ils atteignent la position de fermeture par un mouvement de fermeture par lequel le premier et le deuxième élément de maintien sont déplacés parallèlement ou presque parallèlement l'un à l'autre,
le premier (11 ; 31) et le deuxième (12 ; 32) élément de maintien présentent respectivement un aimant permanent (31a ; 32a) qui présente un axe magnétique (M1 ; M2) s'étendant dans une direction polaire nord-sud magnétique et le mouvement de fermeture (A) se déroule perpendiculairement à l'axe magnétique dès lors que la position de fermeture (P2) a été atteinte ou le mouvement de fermeture (A) se déroule par rapport à l'axe magnétique selon un angle α dès lors que la position de fermeture a été atteinte, dans lequel 70° <= α < 90°, et
le groupe des éléments de maintien présente un troisième (13 ; 33) élément de maintien à action magnétique qui présente un aimant permanent (33a) et est agencé sur le coffret (4 ; 24) ou sur la porte de coffret et qui produit avec le premier (11 ; 31) et le deuxième (12 ; 32) élément de maintien la force magnétique avec laquelle la porte de coffret est maintenue contre le coffret (4 ; 24) dans la position de fermeture, dans lequel le premier (11 ; 31) et le troisième (13 ; 33) sont espacés l'un de l'autre et agencés avec des surfaces extérieures se déroulant parallèlement l'une à l'autre et opposées l'une à l'autre dans la position de fermeture (P2 ; P2') de sorte qu'un espace libre soit formé entre elles, dans lequel le deuxième (12 ; 32) élément de maintien vient en prise dans la position de fermeture ou dans lequel le deuxième (12 ; 32) et le troisième (13 ; 33) élément de maintien sont espacés l'un de l'autre et agencés avec des surfaces extérieures se déroulant parallèlement l'une à l'autre et opposées l'une à l'autre dans la position de fermeture (P2 ; P2') de sorte qu'un espace libre soit formé entre elles, dans lequel le premier (11 ; 31) élément de maintien vient en prise dans la position de fermeture (P2 ; P2').

2. Dispositif d'armoire de laboratoire selon la revendication 1, dans lequel le premier (11 ; 31) et le deuxième (12 ; 32) élément de maintien sont agencés l'un par rapport à l'autre de sorte que, lors de la fermeture de la porte de coffret, ils atteignent d'abord une première position relative (P1) et atteignent ensuite la position de fermeture dans laquelle se présente une deuxième position relative (P2), et
le premier et le deuxième élément de maintien exercent l'un sur l'autre dans la première position relative (P1) une première force magnétique d'attraction (F1) et dans la deuxième position relative (P2) une deuxième force magnétique d'attraction (F2) qui est supérieure à la première force magnétique.

3. Dispositif d'armoire de laboratoire selon la revendication 2, dans lequel le premier (11 ; 31) et le (12 ; 32) deuxième élément de maintien exercent l'un sur l'autre dans une troisième position relative (P3) une troisième force magnétique d'attraction (F3) qui est supérieure à la deuxième force magnétique, et le dispositif d'armoire de laboratoire présente un élément élastique (6) qui dans la position de fermeture est comprimé par la porte de coffret au moyen d'une force magnétique, dans lequel l'élément élastique (6) dans la troisième position relative (P3) est comprimé plus fortement que dans la deuxième position relative.

4. Dispositif d'armoire de laboratoire selon la revendication 3, dans lequel le premier (11 ; 31) et le (12 ; 32) deuxième élément de maintien présentent le même espacement prédéfini dans la deuxième (P2) et la troisième (P3) position relative, et la troisième force magnétique est la force magnétique maximale pouvant être atteinte dans le cadre de cet espacement entre le premier (11 ; 31) et le deuxième (12 ; 32) élément de maintien.

5. Dispositif d'armoire de laboratoire selon l'une des revendications précédentes, dans lequel le groupe des éléments de maintien présente au moins un aimant permanent en forme de parallélépipède rectangle dont la direction polaire nord-sud s'étend perpendiculairement à la surface la plus grande de l'aimant permanent en forme de parallélépipède rectangle.

6. Dispositif d'armoire de laboratoire selon l'une des revendications précédentes, dans lequel le groupe des éléments de maintien présente au moins un aimant permanent en forme d'anneau ou en forme de cylindre creux dont la direction polaire nord-sud s'étend perpendiculairement à l'axe central se déroulant à travers l'ouverture de l'aimant permanent en forme d'anneau ou en forme de cylindre creux.

7. Dispositif d'armoire de laboratoire selon la revendication 5 ou 6, dans lequel le au moins un aimant permanent fait partie d'un élément de maintien du groupe d'éléments de maintien, lequel élément de maintien présente un cadre par lequel au moins un aimant permanent est encadré, dans lequel, lors de la fermeture de la porte de coffret, ce cadre n'est pas sollicité avec une pression par un autre élément de maintien.

8. Dispositif d'armoire de laboratoire selon l'une des revendications précédentes, qui est une armoire de conditionnement en température pour conditionner la température d'échantillons de laboratoire, en particulier un incubateur pour cultures cellulaires.

9. Dispositif d'armoire de laboratoire selon l'une des revendications précédentes, dans lequel le coffret (4 ; 24) du dispositif d'armoire de laboratoire est un coffret extérieur dont les parois de coffret sont en contact avec l'environnement du dispositif d'armoire de laboratoire.

10. Dispositif d'armoire de laboratoire selon l'une des revendications précédentes 1 à 5, dans lequel le coffret (4 ; 24) du dispositif d'armoire de laboratoire est un coffret intérieur se situant à l'intérieur d'un coffret extérieur, dans lequel les parois de coffret du coffret extérieur sont en contact avec l'environnement du dispositif d'armoire de laboratoire.

11. Dispositif d'armoire de laboratoire selon l'une des revendications précédentes, dans lequel un aimant permanent de l'équipement de maintien présente un alliage samarium-cobalt ou est un aimant en fer-néodyme.

12. Dispositif d'armoire de laboratoire selon l'une des revendications précédentes 1 à 6, dans lequel un élément de maintien (11, 12, 13 ; 31, 32, 33) de l'équipement de maintien présente un cadre (31b ; 32b ; 33b) par lequel un aimant permanent de l'élément de maintien est partiellement ou entièrement encadré.
